(19) Europäisches Patentamt
European Patent Office
Office européen des brevets

(11) Veröffentlichungsnummer: **0 000 752**
**B1**

# (12) EUROPÄISCHE PATENTSCHRIFT

(45) Veröffentlichungstag der Patentschrift: **14.01.81**

(21) Anmeldenummer: **78100538.4**

(22) Anmeldetag: **28.07.78**

(51) Int. Cl.³: **C 07 D 249/08**, C 07 D 233/60, C 07 D 231/12, C 07 D 257/04, A 01 N 43/64, A 01 N 41/02

(54) **Alpha-azolysulfide, -sulfoxide und -sulfone, deren Salze und Metallkomplexe, Verfahren zu ihrer Herstellung, sowie sie enthaltende Mittel zur Pilzbekämpfung.**

(30) Priorität: **05.08.77 DE 2735314**

(43) Veröffentlichungstag der Anmeldung: **21.02.79 Patentblatt 79/4**

(45) Bekanntmachung des Hinweises auf die Patenterteilung: **14.01.81 Patentblatt 81/2**

(84) Benannte Vertragsstaaten: **BE CH DE FR GB LU NL SE**

(56) Entgegenhaltungen: **Keine**

(73) Patentinhaber: **BASF Aktiengesellschaft, Carl-Bosch-Strasse 38, D-6700 Ludwigshafen (DE)**

(72) Erfinder: **Sauter, Hubert, Dr., Goethestrasse 23, D-6700 Ludwigshafen (DE)**
Erfinder: **Pommer, Ernst-Heinrich, Dr., Berliner Platz 7, D-6703 Limburgerhof (DE)**
Erfinder: **Zeeh, Bernd, Dr., Thorwaldsenstrasse 5, D-6700 Ludwigshafen (DE)**
Erfinder: **Rentzea, Costin, Dr., Neuenheimer Landstrasse 72, D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



BUNDESDRUCKEREI BERLIN

## Alpha-azolylsulfide, -sulfoxide und -sulfone, deren Salze und Metallkomplexe, Verfahren zu ihrer Herstellung sowie sie enthaltende Mittel zur Pilzbekämpfung

Die Erfindung betrifft neue α-Azolylsulfide, α-Azolyl-sulfoxide und α-Azolyl-sulfone sowie deren Salze und Metallkomplexe, Verfahren zu ihrer Herstellung sowie sie enthaltende Mittel zur Bekämpfung von Pilzen.

Es ist bekannt, daß Imidazol-Derivate, zum Beispiel das 1-[2,4-Dichlorphenyl-β-allyläthyläther]-imidazol (DE-OS 20 63 857), eine gute fungizide Wirksamkeit zeigen. Die Wirkung ist bei niedrigen Aufwandmengen und Anwendungskonzentrationen nicht immer befriedigend. Darüber hinaus ist die fungitoxische Wirkung oft mit einer hohen Phytotoxizität verbunden, so daß in den für die Bekämpfung von Rostpilzen notwendigen Konzentrationen auch die Kulturpflanzen geschädigt werden. Aus diesen Gründen sind sie für den Gebrauch als Pflanzenschutzmittel zur Bekämpfung von Pilzen nicht immer und nicht bei allen Pflanzenarten geeignet.

Es wurde gefunden, daß die neuen α-Azolylsulfide, -sulfoxide und -sulfone der Formel

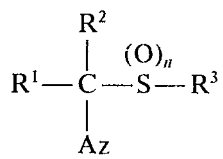

(I)

in der

$R^1$ Wasserstoff, Alkyl, Alkoxycarbonyl oder gegebenenfalls substituiertes Aryl,
$R^2$ Wasserstoff oder Alkyl,
$R^3$ Alkyl, Alkenyl, Alkinyl, gegebenenfalls substituiertes Phenyl oder gegebenenfalls substituiertes Aralkyl,
Az Imidazol-1-yl, Pyrazol-1-yl, 1,2,4-Triazol-1-yl, 1,2,4-Triazol-4-yl, Tetrazol-1-yl oder Tetrazol-2-yl und
n 0, 1 oder 2 bedeuten und deren Salze und Metallkomplexe gut wirksam gegen Schadpilze, insbesondere aus der Klasse der Ascomyceten und Basidiomyceten sind.

$R^1$ bedeutet beispielsweise Wasserstoff, Methyl, Ethyl, n-Propyl, Isopropyl, n-Butyl, n-Pentyl, n-Hexyl, n-Heptyl(3), Methoxycarbonyl, Phenyl, 4-Nitrophenyl, 4-Bromphenyl, 4-Cyanphenyl, 2-Methylphenyl, 4-t-Butylphenyl, 3-Trifluormethylphenyl, 4-Trifluormethylphenyl, 4-Fluorphenyl, 2-Chlorphenyl, 4-Chlorphenyl, 2,4-Dichlorphenyl, 2,6-Dichlorphenyl, 3,4-Dichlorphenyl, -Naphthyl.

$R^2$ bedeutet beispielsweise Wasserstoff, Methyl, n-Propyl.

$R^3$ bedeutet beispielsweise Methyl, Ethyl, n-Propyl, Isopropyl, n-Butyl, t-Butyl, n-Pentyl, n-Hexyl, Allyl, Propargyl, Phenyl, 4-Methoxyphenyl, 4-Tolyl, 4-Chlorphenyl, 3,4-Dichlorphenyl, 2,4-Dichlorphenyl, 2,3,6-Trichlorphenyl, Benzyl, 3-Trifluormethylbenzyl, 4-Chlorbenzyl, 4-Brombenzyl, 3,4-Dichlorbenzyl, 2,4-Dichlorbenzyl, 2,3,6-Trichlorbenzyl, 2-Phenylethyl.

Salze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß die Wahl des Anions beliebig ist.

Metallkomplexe sind Verbindungen der Formel

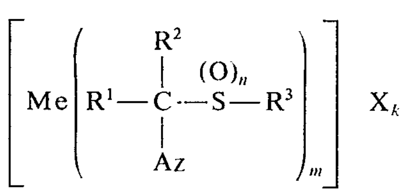

(II)

in der

$R^1$, $R^2$, $R^3$, Az und n die oben angegebene Bedeutung haben und
Me ein Metall, z. B. Kupfer, Zink, Zinn, Mangan, Eisen, Cobalt oder Nickel bedeutet,
X das Anion einer anorganischen Säure bedeutet, z. B. Salzsäure, Schwefelsäure, Phosphorsäure, Bromwasserstoffsäure,
m und k 1, 2, 3 oder 4 bedeuten.

Weiterhin wurde gefunden, daß man α-Azolylsulfide der Formel I (mit n=0) erhält, wenn man α-Chlorsulfide der Formel

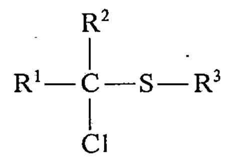

(III)

in welcher
$R^1$, $R^2$ und $R^3$ die oben angegebenen Bedeutungen haben, mit den Azolen H—Az, in denen Az die oben angegebene Bedeutung hat, gegebenenfalls in Gegenwart einer Base und gegebenenfalls in Gegenwart eines Verdünnungsmittels umsetzt. Durch Oxidation der so erhaltenen α-Azolylsulfide der Formel I (n=0) erhält man die α-Azolyl-sulfoxide der Formel I (n=1) und die α-Azolyl-sulfone der Formel I (n=2).

Zur Herstellung der α-Azolylsulfide der Formel I (n=0) ist es zweckmäßig, die α-Chlorsulfide der Formel III ohne Verdünnungsmittel oder in Gegenwart eines Verdünnungsmittels mit etwa 0,5 bis 2 Äquivalenten eines Alkalisalzes des jeweiligen Azols oder mit etwa 0,5 bis 4 Äquivalenten des jeweiligen Azols, gegebenenfalls unter Zusatz von 0,5 bis 4 Äquivalenten einer Base bei Temperaturen von etwa 0 bis 200°C, vorzugsweise +20 bis +160°C in homogener oder inhomogener Phase umzusetzen.

Als Verdünnungsmittel können z. B. Methanol, Äthanol, Isopropanol, n-Butanol, Diäthyläther, Tetrahydrofuran, Dioxan, Aceton, Acetonitril, Dimethylformamid, Dimethylsulfoxid, Chloroform, Methylenchlorid oder Toluol verwendet werden. Als Basen können z. B. organische Amine wie Triäthylamin, Pyridin oder anorganische Verbindungen, z. B. Kaliumcarbonat oder Natriumhydroxid verwendet werden.

Die als Ausgangsstoffe verwendeten α-Chlorsulfide III sind z. T. aus der Literatur bekannt oder können nach literaturbekannten Verfahren hergestellt werden, z. B.

a) durch Chlorierung von Sulfiden mit N-Chlorsuccinimid (siehe z. B. B. L. Tuleen und T. B. Stephens, J. Org. Chem., 34, 31 [1969]) nach dem Schema

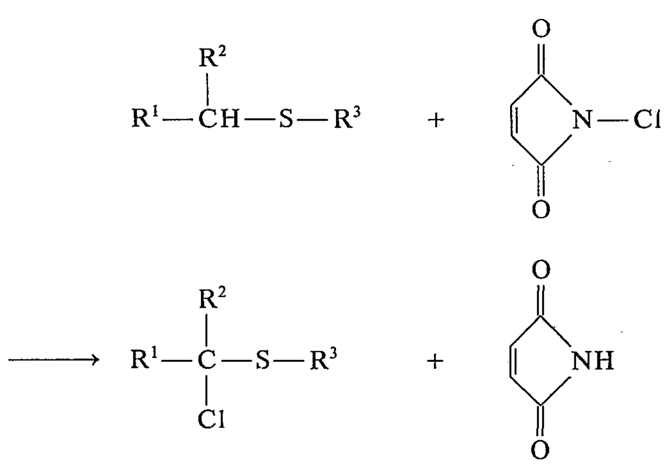

(III)

oder

b) durch Umsetzung von Aldehyden mit Thiolen in Gegenwart von Chlorwasserstoff (siehe z. B. H. Böhme, H. Fischer und R. Frank, Liebigs Ann. Chem., 563, 54 [1949]) nach dem Schema

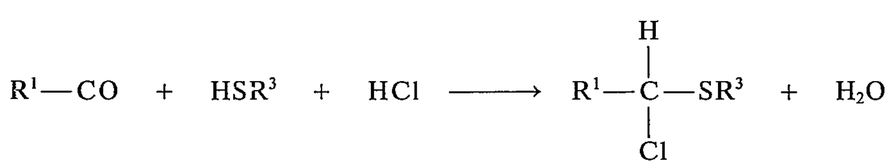

(III)

Zur Herstellung der α-Azolylsulfoxide der Formel I (n=1) setzt man die α-Azolylsulfide gegebenenfalls in Gegenwart eines Verdünnungsmittels mit etwa einem Äquivalent eines geeigneten Oxidationsmittels bei Temperaturen zwischen etwa −30 und +100°C um. Die α-Azolylsulfone (Formel I, n=2) erhält man in ähnlicher Weise bei der Oxidation der α-Azolylsulfide (I, n=0) mit mindestens zwei Äquivalenten eines geeigneten Oxidationsmittels oder bei der Oxidation der α-Azolylsulfoxide (I, n=1) mit mindestens einem Äquivalent eines Oxidationsmittels.

Als Oxidationsmittel können beispielsweise Kaliumpermanganat, Wasserstoffperoxid oder Percarbonsäuren wie Peressigsäure, Perbenzoesäure oder 3-Chlorperbenzoesäure verwendet werden. Als Verdünnungsmittel können z. B. Wasser, Essigsäure, Methanol, Aceton, Chloroform oder Methylenchlorid verwendet werden. Ein bevorzugtes Verfahren zur Herstellung der Sulfoxide (I, n = 1) ist die Umsetzung der Sulfide (I, n = 0) mit einem Äquivalent 3-Chlorperbenzoesäure in Methylenchlorid bei 0 bis 25° C. Ein bevorzugtes Verfahren zur Herstellung der Sulfone ist die Umsetzung der Sulfide (I, n = 0) mit zwei Äquivalenten 3-Chlorperbenzoesäure in Methylenchlorid bei 15 bis 41° C.

Die Verbindungen der Formel I (n = 0, 1, 2) sind in vielen organischen Lösungsmitteln, z. B. in Essigester, Aceton, Äthanol, Methylenchlorid, Chloroform, Dimethylsulfoxid, Dimethylformamid und N-Methylpyrrolidon leicht lösliche Substanzen. Die $\alpha$-Azolylsulfide (I = 0) sind darüber hinaus auch in Toluol gut löslich.

Sie lassen sich mit Säuren in ihre Salze, z. B. Hydrochloride, Sulfate, Nitrate, Oxalate, Formiate, Acetate oder Dodecylbenzylsulfonate überführen.

Ferner wurde gefunden, daß man die Metall-Komplexe der Formel II erhält, wenn man $\alpha$-Azolylsulfide bzw. deren Derivate der Formel I mit Metallsalzen der Formel

$$MeX_k \cdot a\ H_2O \qquad (IV)$$

in welcher
Me, X und k die oben angegebene Bedeutung haben und a 0, 1, 2, 3 und 4 bedeutet, in Gegenwart eines Lösungsmittels umsetzt. Hier steht Me vorzugsweise für Metalle der I., II. und IV. bis VIII. Nebengruppe des Periodensystems der Elemente sowie für Metalle der II. und IV. Hauptgruppe, insbesondere für Kupfer, Zink, Zinn, Mangan, Eisen, Cobalt oder Nickel.

Die Metallsalze der Formel IV sind allgemein bekannte, leicht zugängliche Verbindungen.

Für die Herstellung der Metall-Komplexe der Formel II kommen alle mit Wasser mischbaren Lösungsmittel in Frage. Hierzu gehören vorzugsweise Methanol, Äthanol, Isopropanol, Aceton, Tetrahydrofuran und Dioxan. Dabei arbeitet man im allgemeinen bei Temperaturen zwischen 0 und 100° C, vorzugsweise zwischen 10 und 35° C.

Die Sulfide (I, n = 0) und Sulfone (I, n = 2) enthalten jeweils ein Asymmetrisches Kohlenstoffatom und fallen demgemäß als Enantiomerengemische an, die in die optisch aktiven Verbindungen getrennt werden können. Im Falle der Sulfoxide I (n = 1) treten durch das dem Asymmetrischen Kohlenstoff benachbarte Asymmetrische Schwefelatom Diastereomerengemische auf, die in üblicher Weise, z. B. durch Kristallisation oder Chromatographie in die einzelnen Komponenten getrennt werden können. Für die Anwendung als Fungizide oder als Mittel zur Regulierung des Pflanzenwachstums ist jedoch eine Trennung der Enantiomeren oder Diastereomeren normalerweise nicht erforderlich.

## Beispiel 1

### tert.-Butyl-[(2,4-dichlorphenyl)-1,2,4-triazol-1-yl-methyl]-sulfid (Verbindung Nr. 65)

Zur Lösung von 22,1 g 1,2,4-Triazol in 500 ml wasserfreiem Aceton gibt man 60,5 g tert.-Butyl-[(2,4-dichlorphenyl)-chlormethyl]-sulfid. Nach Zugabe von 44,2 g feingepulvertem Kaliumcarbonat erhitzt man die Mischung unter Rühren sieben Stunden unter Rückfluß. Danach filtriert man die unlöslichen Bestandteile ab, engt das Filtrat im Vakuum zur Trockene ein und versetzt den Rückstand mit 300 ml Wasser. Man extrahiert die wäßrige Phase 3mal mit je 200 ml Methylenchlorid, wäscht die vereinigten Extrakte mit 200 ml Wasser, trocknet sich und engt im Vakuum ein. Nach Zugabe von 100 ml Diisopropyläther gewinnt man aus dem Rückstand 35,6 g (53%) farblose Kristalle vom Schmp. 95 bis 97° C.

$^1H-NMR$ (100 MHz, $CDCl_3$): $\delta = 1,3$ (s, 9 H), 6,95 (s, 1 H), 7,0 – 7,4 (m, 3 H, ABX), 8,0 (s, 1 H), 8,8 ppm (s, 1 H).

## Beispiel 2

### Bis-⟨tert.-butyl-[(2,4-dichlorphenyl)-1,2,4-triazol-1-yl-methyl]-sulfid⟩-kupfer(II)-chlorid

Zur Lösung von 9,5 g tert.-Butyl-[(2,4-dichlorphenyl)-1,2,4-triazol-1-yl-methyl]-sulfid in 100 ml Äthanol tropft man 15 ml einer molaren äthanolischen Lösung von Kupfer(II)-chlorid-dihydrat. Aus dieser tiefblauen Lösung isoliert man nach zweitägigem Stehen bei 0° C 9,8 g blaue Kristalle, die mit Äthanol und Äther gewaschen werden. Schmp. 130° C.

Beispiel 3

1-Pentyl-[1-(1,2,4-triazol-1-yl)-pentyl-(1)]-sulfid (Verbindung Nr. 75)

Zur Suspension von 13,0 g Natriumhydrid in 100 ml Dimethylformamid tropft man unter Rühren eine Lösung von 43,5 g Triazol in 200 ml Dimethylformamid. Nach Beendigung der Wasserstoffentwicklung tropft man zu der auf Raumtemperatur abgekühlten Mischung 97,0 g 1-Pentyl-[1-chlorpentyl-(1)]-sulfid hinzu, wobei sich das Gemisch erwärmt. Anschließend rührt man noch acht Stunden bei 80°C. Die Reaktionsmischung wird sodann im Vakuum eingeengt, mit 500 ml Wasser versetzt und 5mal mit Methylenchlorid extrahiert. Die vereinigten und eingeengten Extrakte chromatographiert man an Kieselgel (5 x 70 cm) zunächst unter Verwendung von Methylenchlorid als Laufmittel. Nach Durchlauf der ersten bräunlichen Zone werden steigende Mengen Aceton zugesetzt (bis 10% Aceton). Als zweite Zone isoliert man so 15,0 g Produkt als blaßgelbes Öl. IR (Film): 2955, 2925, 2860, 1496, 1460, 1271, 1190, 1133, 1008, 677 $cm^{-1}$.

$^1$H—NMR (270 MHz, $CDCl_3$): $\delta$=0,9 (»tr«, 6 H), 1,3 (m, 8 H), 1,5 (m, 2 H), 2,1 (m, 2 H), 2,4 (m, 2 H), 5,4 (tr, 1 H), 8,0 (s, 1 H), 8,4 ppm (s, 1 H).

Beispiel 4

1-Pentyl-[1-(1,2,4-triazol-1-yl)-pentyl-(1)]-sulfon (Verbindung Nr. 71)

Zur Lösung von 10,0 g 85prozentiger 3-Chlorperbenzoesäure in 140 ml Methylchlorid tropft man unter Rühren und Eiskühlung eine Lösung von 6,0 g 1-Pentyl-[1-(1,2,4-triazol-1-yl)-pentyl-(1)]-sulfid. Nach Abklingen der anfänglichen Erwärmung rührt man noch 2 Tage bei 25°C. Die Mischung wird dann mit Natriumcarbonatlösung, Natriumsulfitlösung und Wasser gewaschen und die organische Phase getrocknet. Nach dem Einengen bleibt ein hellgelbes Öl, aus dem beim Anreiben mit Diisopropyläther 3,5 g farblose Kristalle vom Schmp. 69 bis 71°C gewonnen werden.

NMR ($CDCl_3$, 100 MHz): $\delta$=0,9 (m, 6 H), 1,4 (m, 8 H), 1,8 (m, 2 H), 2,5 (m, 2 H), 2,8 (»dd«, 2 H), 5,4 (dd, 1 H), 8,1 (s, 1 H), 5 ppm (s, 1 H).

Beispiel 5

tert.-Butyl-[(2-methylphenyl)-imidazol-1-yl-methyl]-sulfid (Verbindung Nr. 89)

Zur Lösung von 43,6 g Imidazol in 300 ml Aceton tropft man unter Rühren 82 g tert.-Butyl-[(2-methylphenyl)-chlormethyl]-sulfid. Nach Zugabe von 88 g feingepulvertem Kaliumcarbonat erhitzt man 5 Stunden unter Rückfluß. Danach wird von den ungelösten Bestandteilen abfiltriert und das Filtrat eingeengt. Der mit 300 ml Wasser versetzte Rückstand wird mit 3 x 200 ml Methylenchlorid extrahiert. Aus den vereinigten organischen Phasen bleiben nach Waschen mit Wasser, Trocknen und Einengen 69 g eines bräunlichen Öls zurück, die in 1 l Diisopropyläther gelöst werden. Durch tropfenweise Zugabe von 90 ml einer 2,85molaren Lösung von Chlorwasserstoff in Diisopropyläther unter Rühren fallen aus dieser Lösung blaßgelbe Kristalle des Hydrochlorids, die aus Aceton umkristallisiert werden, an. Man erhält so 51 g farbloses tert.-Butyl-[(2-methylphenyl)-imidazol-1-yl-methyl]-sulfidhydrochlorid vom Schmp. 168 bis 170°C (Verbindung Nr. 110).

Nach Übergießen des Hydrochlorids mit einer wäßrigen Lösung von 25 g Natriumhydrogencarbonat extrahiert man die freie Base mit 3 x 200 ml Äther. Nach dem Trocknen und Einengen fällt sie als blaßgelbes Öl (27 g) an, das allmählich durchkristallisiert (Fp. 93—95°).

$^1$H—NMR (60 MHz, $CDCl_3$): $\delta$=1,3 (s, 9 H), 2,4 (s, 3 H), 6,25 (s, 1 H), 6,7—7,1 (m, 6 H), 7,65 ppm (dd, 1 H).

Beispiel 6

4-Chlorphenyl-[(4'-chlorphenyl)-imidazolyl-methyl]-sulfid (Verbindung Nr. 7)

Die Mischung von 60,7 g 4-Chlorphenyl-(chlormethyl-(4'-chlorphenyl))-sulfid, 27,2 g Imidazol und 55,4 g Kaliumcarbonat in 400 ml Aceton wird unter Rühren 5 Stunden unter Rückfluß erhitzt. Danach wird filtriert, das Filtrat zur Trockne eingeengt und mit 500 ml Wasser versetzt. Man extrahiert diese Mischung 3mal mit je 200 ml Methylenchlorid. Die vereinigten Extrakte werden über Natriumsulfat getrocknet. Aus der im Vakuum zur Trockene eingeengten Lösung werden nach Anreiben mit Diisopropyläther 26 g (38%) farblose Kristalle vom Schmp. 88°C isoliert.

$^1$H—NMR (60 MHz, $CDCl_3$): $\delta$=6,3 (s, 1 H), 6,9—7,4 (m, 1 OH), 7,5 (br. s, 1 H).

Beispiel 7
tert.-Butyl-[(2,6-dichlorphenyl)-pyrazolylmethyl]-sulfid (Verbindung Nr. 53)

Zur Lösung von 193 g tert.-Butyl-(chlormethyl-(2,6-dichlorphenyl))-sulfid und 44 g Pyrazol in 1 l Toluol tropft man 66 g Triäthylamin und erhitzt nach Abklingen der schwach exothermen Reaktion noch 1 Stunde unter Rückfluß. Man filtriert vom Ungelösten ab, engt das Filtrat ein und versetzt den Rückstand mit 500 ml Wasser. Nach Extraktion mit 3mal 200 ml Methylenchlorid erhält man beim Einengen der vereinigten Extrakte einen festen Rückstand, der nach dem Waschen mit Diisopropyläther 117 g (57%) farblose Kristalle vom Schmp. 97 – 98° C ergibt.

$^1H$—NMR (60 MHz, $CDCl_3$): $\delta$=1,4 ppm (s, 9 H), 6,2 (»tr.«, 1 H), 7,0 – 7,3 (m, 3 H), 7,4 (»d«, 1 H), 8,2 (»d«, 2 H).

Beispiel 8
2,4-Dichlorbenzyl-(1,2,4-triazolylmethyl)-sulfid (Verbindung Nr. 78)

Eine Mischung von 60,4 g Chlormethyl-(2,4-dichlorbenzyl)-sulfid, 35 g 1,2,4-Triazol und 69 g gepulvertem Kaliumcarbonat in 300 ml wasserfreiem Aceton wird 10 Stunden unter Rückfluß zum Sieden erhitzt. Danach filtriert man die unlöslichen Bestandteile ab, engt das Filtrat im Vakuum zur Trockne ein und versetzt den Rückstand mit 300 ml Wasser. Dann wird 3mal mit je 200 ml Methylenchlorid extrahiert, die vereinigten Extrakte getrocknet und im Vakuum eingeengt. Aus dem zurückbleibenden braunen Öl werden durch Kristallisation aus Diisopropyläther/Methanol bei –60° C 39,8 g (58%) farblose Kristalle vom Schmp. 68 – 70° C gewonnen.

$^1H$—NMR (60 MHz, $CDCl_3$): $\delta$=3,8 (s, 2 H), 5,0 (s, 2 H), 7,0 – 7,5 (m, 3 H), 7,9 (s, 1 H), 8,2 (s, 1 H).

Beispiel 9
2,4-Dichlorbenzyl-(2',4'-dichlorphenyl-1,2,4-triazol-1-yl-methyl)-sulfid (Verbindung Nr. 61) und 2,4-Dichlorbenzyl-[(2',4'-dichlorphenyl-(1,2,4-triazol-4-yl)-methyl]-sulfid (Verbindung Nr. 64)

Eine Mischung von 200 g 2,4-Dichlorbenzyl-[(2',4'-dichlorphenyl)-chlormethyl]-sulfid, 145 g 1,2,4-Triazol und 138 g gepulvertem Kaliumcarbonat in 2 l wasserfreiem Aceton wird unter kräftigem Rühren 9 Stunden unter Rückfluß erhitzt. Danach werden die festen Bestandteile durch Filtration entfernt, das Filtrat im Vakuum zur Trockne eingeengt und der ölige Rückstand nach Zusatz von 500 ml Wasser fünfmal mit je 200 ml Methylenchlorid extrahiert. Die vereinigten organischen Phasen werden über Natriumsulfat getrocknet und dann im Vakuum zur Trockne eingeengt. Beim Anreiben des Rückstandes mit Diisopropyläther erhält man 157 g farblose Kristalle, die die isomeren Triazolylverbindungen im Verhältnis 8 : 2 enthalten.

Dieses Gemisch wird mehrfach mit heißem Hexan extrahiert. Aus den vereinigten Hexanlösungen erhält man beim Abziehen des Lösungsmittels im Vakuum 122 g (65%), 2,4-Dichlorbenzyl-[(2',4'-dichlorphenyl)-1,2,4-triazol-1-yl-methyl]-sulfid in Form farbloser Kristalle vom Schmp. 120 – 125° C.

$^1H$—NMR (220 MHz, $CDCl_3$): $\delta$=3,9 ppm (breites s, 2 H), 6,65 (s, 1 H), 7,0 – 7,5 (m, 6 H), 8,0 (s, 1 H), 8,6 (s, 1 H).

Der farblose, unlösliche Rückstand der Hexanextraktionen besteht aus 27 g (12%) 2,4-Dichlorbenzyl-[(2',4'-dichlorphenyl)-(1,2,4-triazol-4-yl)-methyl]-sulfid vom Schmp. 132 – 133° C.

$^1H$—NMR (220 MHz, $CDCl_3$): $\delta$=3,9 ppm (s, 2 H), 6,5 (s, 1 H), 7,0 – 7,5 (m, 6 H), 8,45 (s, 2 H).

Beispiel 10
tert.-Butyl-[(4-chlorphenyl)-1,2,4-triazol-1-yl-methyl]-sulfid (Verbindung Nr. 30)

Zu einer Lösung von 78 g tert.-Butyl-[(4-chlorphenyl)-chlormethyl]-sulfid in 175 ml trockenem Dimethylformamid (DMF) tropft man unter Rühren 350 ml einer molaren Lösung 1,2,4-Triazolylnatrium in DMF (hergestellt aus 0,35 Mol Natriumhydrid und 0,35 Mol 1,2,4-Triazol in 350 ml DMF). Nach Abklingen der schwach exothermen Reaktion rührt man noch 4 Stunden bei 80° C nach. Der Ansatz wird in 1 l Wasser gegossen und mit 3mal 200 ml Methylenchlorid extrahiert. Die vereinigten organischen Phasen werden mit Wasser gewaschen, über Natriumsulfat getrocknet und im Vakuum

eingedampft. Der Rückstand bildet beim Verreiben mit 150 ml Diisopropyläther 40,0 g (46%) farblose Kristalle vom Schmp. 95° C.

$^1H-NMR$ (60 MHz, $CDCl_3$): $\delta=1,3$ ppm (s, 9 H), 6,6 (s, 1 H), 7,2 (AA'BB', 4 H), 8,0 (s, 1 H), 8,7 (s, 1 H).

### Beispiel 11
### tert.-Butyl-((4-chlorphenyl)-1,2,4-triazolylmethyl)-sulfoxid (Verbindung Nr. 31)

Zur Lösung von 9,7 g tert.-Butyl-[(4-chlorphenyl)-1,2,4-triazolylmethyl]-sulfid in 20 ml Methylenchlorid tropft man unter Eiskühlung eine Lösung von 7,1 g 85prozentiger 3-Chlorperbenzoesäure in 70 ml Methylenchlorid. Man rührt noch 2 Stunden bei 0° C nach bis laut Dünnschichtchromatogramm ($SiO_2$, Methylenchlorid/Aceton 7 : 3) kein Sulfid ($R_F=0,57$) mehr vorhanden ist und nur noch die beiden diastereomeren Sulfoxide erkennbar sind ($R_F=0,38$ und 0,20). Die Lösung wird sodann bis zur Beendigung der $CO_2$-Entwicklung mit wäßriger Natriumhydrogencarbonatlösung gewaschen. Nach dem Waschen der organischen Phase mit Wasser wird diese getrocknet, eingeengt und der feste Rückstand mit wenig Äther gewaschen. Man erhält 7,0 g (67%) farblose Kristalle vom Schmp. 125–128° C.

$^1H-NMR$ (220 MHZ, $CDCl_3$): $\delta=1,10$ und 1,15 (zwei s, zus. 9 H), 6,18 und 6,24 (zwei s, zus. 1 H), 7,3–7,7 (m, 4 H), 8,01 und 8,07 (zwei s, zus. 1 H), 8,41 und 8,51 (zwei s, zus. 1 H).

### Beispiel 12
### tert.-Butyl-[(4-chlorphenyl)-1,2,4-triazolylmethyl)]-sulfon (Verbindung Nr. 32)

Zur Lösung von 10,7 g tert.-Butyl-[(4-chlorphenyl)-1,2,4-triazolylmethyl]-sulfoxid in 50 ml Methylenchlorid tropft man bei 20° C eine Lösung von 7,3 g 85prozentiger 3-Chlorperbenzoesäure in 100 ml Methylenchlorid zu. Nach dem Ergebnis der Dünnschichtchromatographie (DC) wird das weniger polare Sulfoxid schneller oxidiert; erst durch längeres Nachrühren bei Raumtemperatur wird auch das stärker polare Sulfoxid oxidiert. Nach 18 Stunden ist nach DC ($SiO_2$, Methylenchlorid/Aceton 7 : 3) die Umsetzung beendet. Das Sulfon ($R_F=0,46$) wird durch Waschen der Lösung mit Natriumhydrogencarbonatlösung und Wasser, Trocknen und Einengen der organischen Phase isoliert. Nach dem Waschen des Rückstandes der organischen Phase isoliert. Nach dem Waschen des Rückstandes mit Diisopropyläther erhält man 8,7 g (77%) farblose Kristalle vom Schmp. 150° C.

$^1H-NMR$ (220 MHz, $CDCl_3$): $\delta=1,3$ ppm (s, 9 H), 6,75 (s, 1 H), 7,5 (AA'BB', 4 H), 8,03 (s, 1 H), 8,84 (s, 1 H).

### Beispiel 13
### 4-Chlorphenyl-[(4'-chlorphenyl)-(tetrazol-1-yl)-methyl]-sulfid und 4-Chlorphenyl-[(4'-chlorphenyl)-(tetrazol-2-yl)-methyl]-sulfid (Verbindung Nr. 15)

Zu einer Mischung von 26,1 g Tetrazol und 102 g 4-Chlorphenyl-[(4'-chlorphenyl)-chlormethyl]-sulfid in 3,5 l Toluol tropft man bei Raumtemperatur 37,4 g Triäthylamin unter Rühren zu. Die Mischung wird danach 4 Stunden unter Rückfluß erhitzt. Man filtriert vom Unlöslichen ab, wäscht das Filtrat mit Wasser und trocknet die organische Phase über Natriumsulfat. Nach Abziehen des Lösungsmittels im Vakuum bleibt ein fester Rückstand, aus dem nach Zusatz von 50 ml Diisopropyläther 74,7 g (67%) gelbstichige Kristalle vom Schmp. 104–106° C isoliert werden.

$^1H-NMR$ (60 MHz, $CDCl_3$): $\delta=6,7-7,6$ ppm (m, 9 H), 8,4 und 8,9 (zwei s, zus. 2 H).

Beispiele für die erfindungsgemäßen Verbindungen der Formel I seien in folgender Tabelle 1 genannt:

Tabelle 1

| Verb.**) | $R^1$ | $R^2$ | Az (Salz) | n | $R^3$ | Schmp. (°C) |
|---|---|---|---|---|---|---|
| 1 | 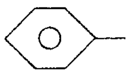 | H | 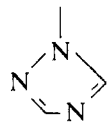 | 0 | 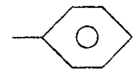 | 132–134 |
| 2 | $H_3C$— | H | 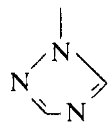 | 0 | 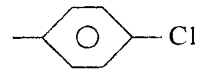 | IR (Film):<br>1495, 1470, 1270, 1192, 1131, 1088, 1007, 817, 666 $cm^{-1}$ |
| 3 | 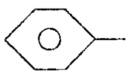 | H | 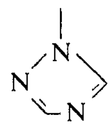 | 0 | 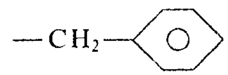 | IR (Film):<br>3060, 3020, 1492, 1450, 1271, 1190, 1131, 1008, 695, 677, 658 $cm^{-1}$ |
| 4 | 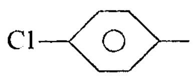 | H | 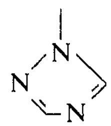 | 0 | 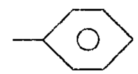 | 98 |
| 5 | 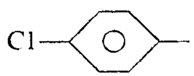 | H | 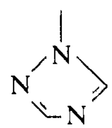 | 1 | 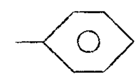 | 131–139*) |
| 6 | 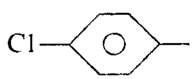 | H | 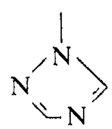 | 2 | 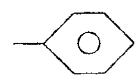 | 215 |

*) Diastereomerengemisch der Sulfoxide
**) Die Strukturen aller aufgeführten Verbindungen wurden durch $^1$H-NMR-Spektren bestätigt

(Fortsetzung)

| Verb.**) | $R^1$ | $R^2$ | Az (Salz) | n | $R^3$ | Schmp. ( C) |
|---|---|---|---|---|---|---|
| 7 | 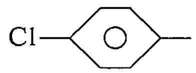 | H | 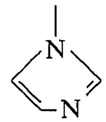 | 0 | 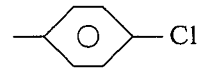 | 88 |
| 8 | 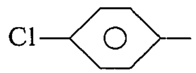 | H | 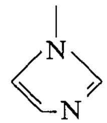 · $(COOH)_2$ | 0 | 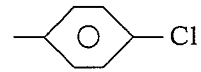 | 85–87 |
| 9 | 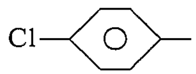 | H | 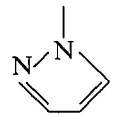 | 0 | 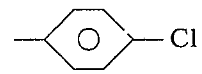 | 62–64 |
| 10 | 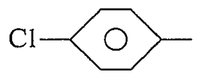 | H | 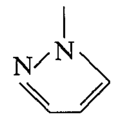 | 1 | 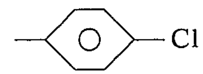 | 116–118*) |
| 11 | 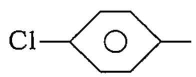 | H | 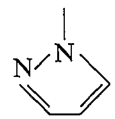 | 2 | 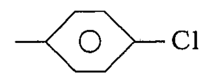 | 147–150 |
| 12 | 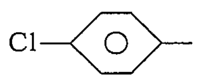 | H | 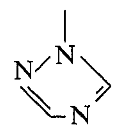 | 0 | 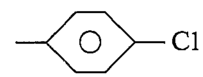 | 136–138 |
| 13 | 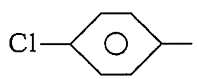 | H | 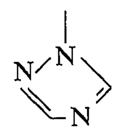 | 1 | 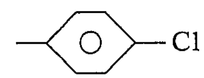 | 147***) |

*) Diastereomerengemisch der Sulfoxide
**) Die Strukturen aller aufgeführten Verbindungen wurden durch $^1H$-NMR-Spektren bestätigt
***) Gemisch von Tetrazolisomeren

(Fortsetzung)

| Verb. | $R^1$ | $R^2$ | Az (Salz) | n | $R^3$ | Schmp. (°C) |
|---|---|---|---|---|---|---|
| 14 | 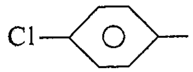 | H | 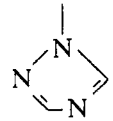 | 2 | 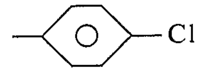 | 204 |
| 15 | 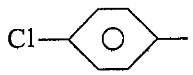 | H | 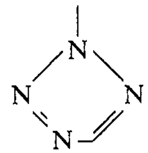 + 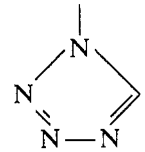 *) | 0 | 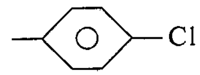 | 104–106 |
| 16 | 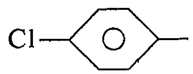 | H | 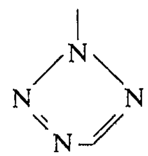 + 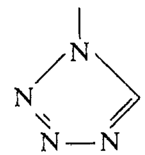 *) | 1 | 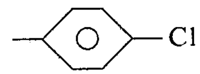 | 110–112**) |
| 17 | 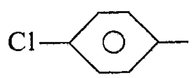 | H | 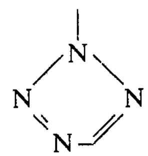 + 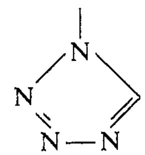 *) | 2 | 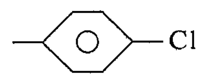 | 123–125 |
| 18 | 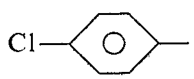 | H | 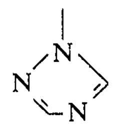 | 0 | 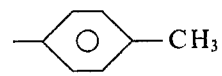 | 130 |
| 19 | 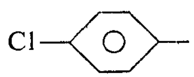 | H | 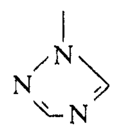 | 1 | 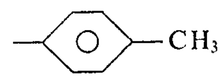 | 147–149**) |

*) Gemisch von Tetrazolisomeren
**) Diastereomerengemisch der Sulfoxide

(Fortsetzung)

| Verb. | $R^1$ | $R^2$ | Az (Salz) | n | $R^3$ | Schmp. (°C) |
|---|---|---|---|---|---|---|
| 20 | 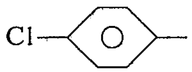 | H | 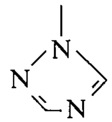 | 1 | 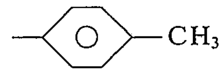 | 154–155*) |
| 21 | 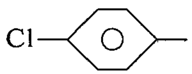 | H | 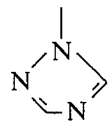 | 2 | 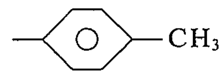 | 245 |
| 22 | 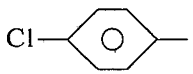 | H | 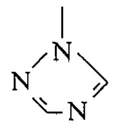 | 0 | 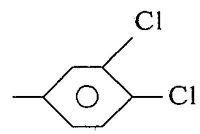 | 102 |
| 23 | 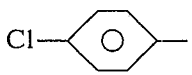 | H | 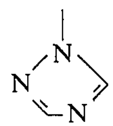 | 0 | 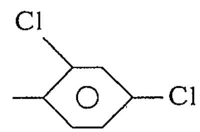 | 135–137 |
| 24 | 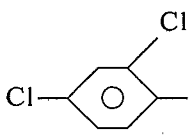 | H | 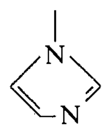 | 0 | tert.-Butyl | IR (Film): 2950, 1581, 1460, 1362, 1212, 1152, 1096, 1064, 1043, 844, 787, 657 $cm^{-1}$ |
| 25 | 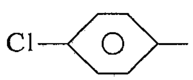 | H | 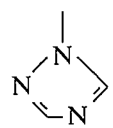 | 0 | 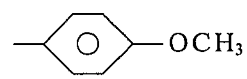 | 116 |

*) Stärker polares Sulfoxid, durch Kristallisation abgetrennt aus dem Diastereomerengemisch Nr. 19

(Fortsetzung)

| Verb. | $R^1$ | $R^2$ | Az (Salz) | n | $R^3$ | Schmp. (°C) |
|---|---|---|---|---|---|---|
| 26 | 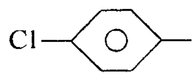 | H | 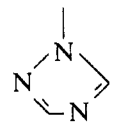 | 2 | 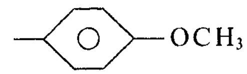 | 198 |
| 27 | 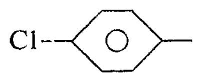 | H | 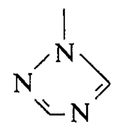 | 0 | 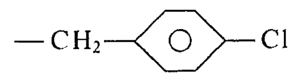 | 57–58 |
| 28 | 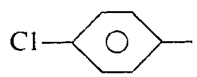 | H | 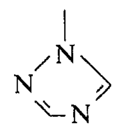 | 0 | Äthyl | IR (Film): 2965, 2925, 1490, 1271, 1131, 1088, 1010, 675 $cm^{-1}$ |
| 29 | 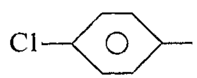 | H | 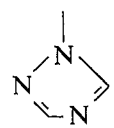 | 0 | n-Butyl | IR (Film): 2945, 2920, 1486, 1268, 1127, 1084, 1007, 671 $cm^{-1}$ |
| 30 | 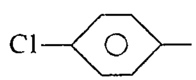 | H | 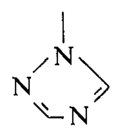 | 0 | tert.-Butyl | 95 |
| 31 | 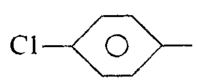 | H | 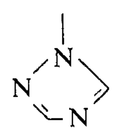 | 1 | tert.-Butyl | 125–128*) |

*) Diastereomerengemisch

(Fortsetzung)

| Verb. | $R^1$ | $R^2$ | Az (Salz) | n | $R^3$ | Schmp. ( C) |
|---|---|---|---|---|---|---|
| 32 | 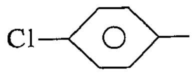 | H | 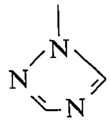 | 2 | tert.-Butyl | 150 |
| 33 | 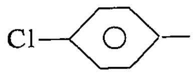 | H | 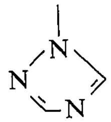 | 0 | Allyl | IR (Film):<br>3054, 1489, 1403, 1270,<br>1130, 1087, 1007,<br>672 $cm^{-1}$ |
| 34 | 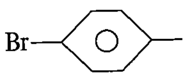 | H | 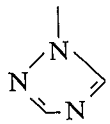 | 0 | tert.-Butyl | 77–78 |
| 35 | 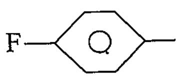 | H | 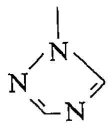 | 0 | tert.-Butyl | 102–104 |
| 36 | 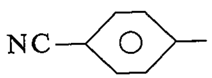 | H | 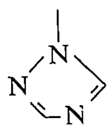 | 0 | tert.-Butyl | 73–75 |
| 37 | 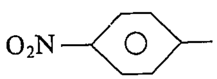 | H | 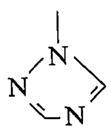 | 0 | tert.-Butyl | 76–78 |
| 38 | 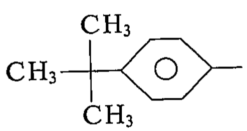 | H | 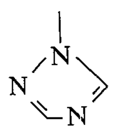 | 0 | tert.-Butyl | 94 |

(Fortsetzung)

| Verb. | $R^1$ | $R^2$ | Az (Salz) | n | $R^3$ | Schmp. (°C) |
|---|---|---|---|---|---|---|
| 39 | H | H | 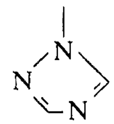 | 0 | 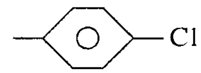 | 47–49 |
| 40 | 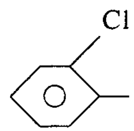 | H | 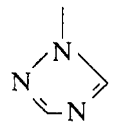 | 0 | 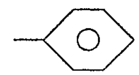 | 91 |
| 41 | 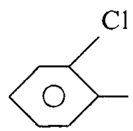 | H | 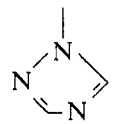 | 1 | 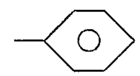 | 70–88*) |
| 42 | 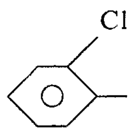 | H | 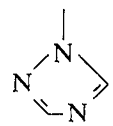 | 2 | 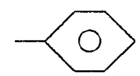 | 143 |
| 43 | 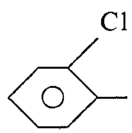 | H | 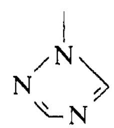 | 0 | tert.-Butyl | 84 |
| 44 | 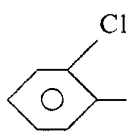 | H | 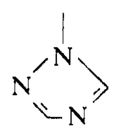 | 1 | tert.-Butyl | 154–157*) |

*) Diastereomerengemisch

(Fortsetzung)

| Verb. | $R^1$ | $R^2$ | Az (Salz) | n | $R^3$ | Schmp. (°C) |
|---|---|---|---|---|---|---|
| 45 | 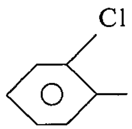 | H | 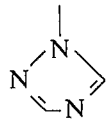 | 2 | tert.-Butyl | 126 |
| 46 | 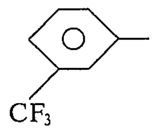 | H | 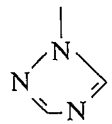 | 0 | 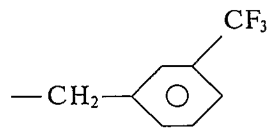 | IR (Film): 1445, 1323, 1270, 1160, 1115, 1070, 790, 695, 660 $cm^{-1}$ |
| 47 | 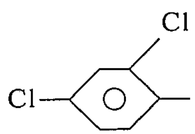 | H | 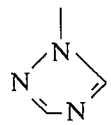 · $(COOH)_2$ | 0 | 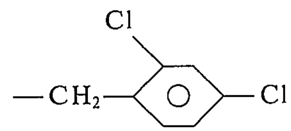 | 133–135 |
| 48 | 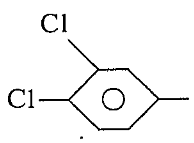 | H | 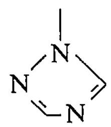 | 0 | 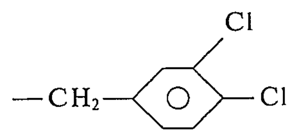 | 101 |
| 49 | 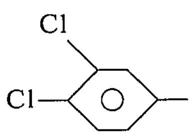 | H | 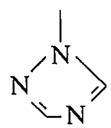 | 2 | 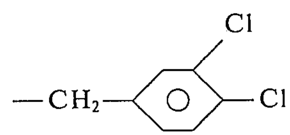 | 195–197 |
| 50 | 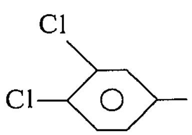 | H | 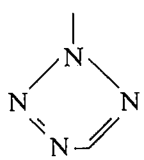 + 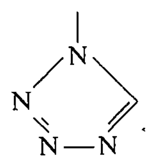 | 0 | 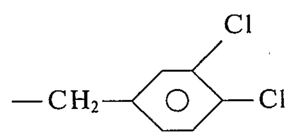 | 82–84 |

(Fortsetzung)

| Verb. | $R^1$ | $R^2$ | Az (Salz) | n | $R^3$ | Schmp. (°C) |
|---|---|---|---|---|---|---|
| 51 | 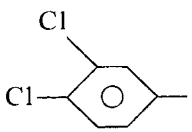 | H | 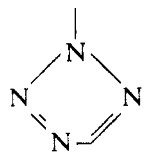 + 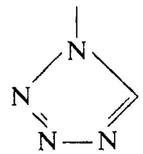 | 1 | 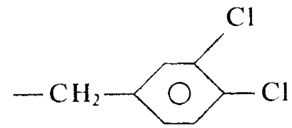 | 104–110*) |
| 52 | 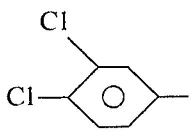 | H | 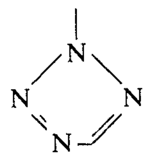 + 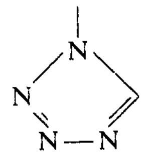 | 2 | 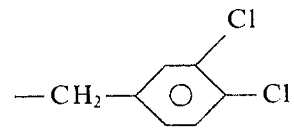 | 100–105 |
| 53 | 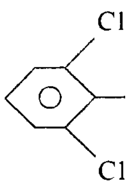 | H | 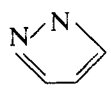 | 0 | tert.-Butyl | 97–98 |
| 54 | 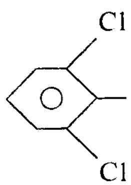 | H | 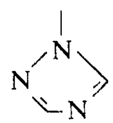 | 0 | tert.-Butyl | 110–112 |
| 55 | 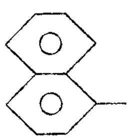 | H | 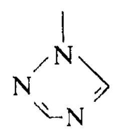 | 0 | tert.-Butyl | 112–115 |

*) Diastereomerengemisch

(Fortsetzung)

| Verb. | $R^1$ | $R^2$ | Az (Salz) | n | $R^3$ | Schmp. ( C) |
|---|---|---|---|---|---|---|
| 56 | 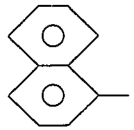 | H | 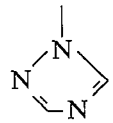 | 1 | tert.-Butyl | 122–127*) |
| 57 | 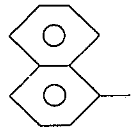 | H | 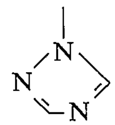 | 2 | tert.-Butyl | 180–183 |
| 58 | 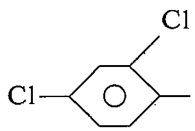 | H | 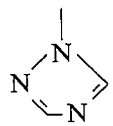 | 0 | 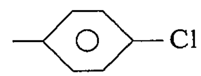 | 128 |
| 59 | 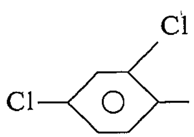 | H | 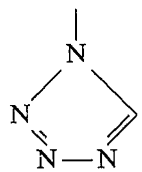 | 0 | 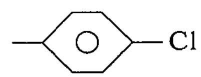 | 148–150 |
| 60 | 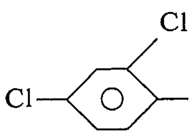 | H | 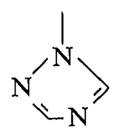 | 0 | 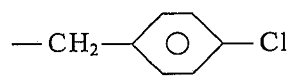 | 105 |
| 61 | 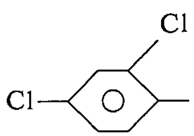 | H | 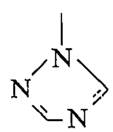 | 0 | 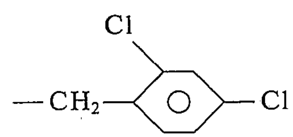 | 120–125 |

*) Diastereomerengemisch

(Fortsetzung)

| Verb. | $R^1$ | $R^2$ | Az (Salz) | n | $R^3$ | Schmp. (°C) |
|---|---|---|---|---|---|---|
| 62 | 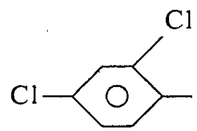 | H | 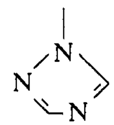 | 1 | 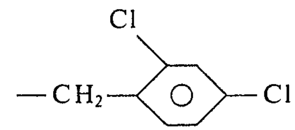 | 125–131*) |
| 63 | 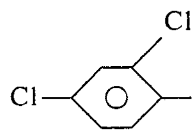 | H | 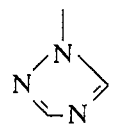 | 2 | 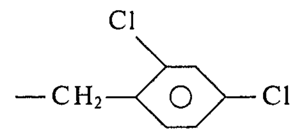 | 163–165 |
| 64 | 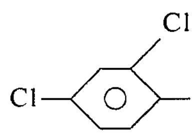 | H | 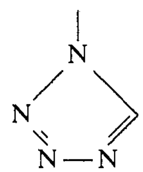 | 0 | 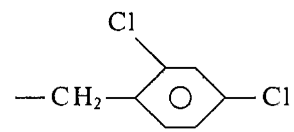 | 132–133 |
| 65 | 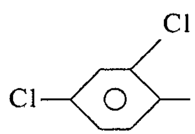 | H | 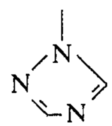 | 0 | tert.-Butyl | 95–97 |
| 66 | 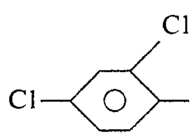 | H | 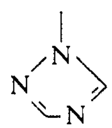 | 0 | $-CH_3$ | 66–88 |
| 67 | 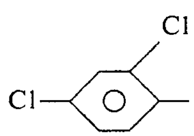 | H | 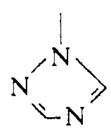 | 0 | 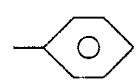 | 95–97 |

*) Diastereomerengemisch

(Fortsetzung)

| Verb. | $R^1$ | $R^2$ | Az (Salz) | n | $R^3$ | Schmp. (°C) |
|---|---|---|---|---|---|---|
| 68 | 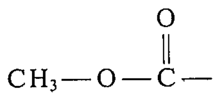 | H | 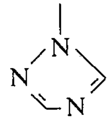 | 0 | 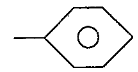 | IR (Film) $cm^{-1}$ 2950, 1748, 1494, 1433, 1260, 1196, 1128, 994, 746, 689, 672 |
| 69 | n-Hexyl | H | 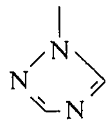 | 0 | 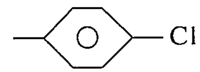 | IR (Film): 2945, 2920, 2850, 1496, 1471, 1270, 1132, 1089, 1008, 818, 674 $cm^{-1}$ |
| 70 | n-Butyl | H | 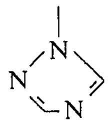 | 0 | 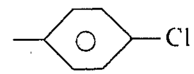 | IR (Film): 2950, 2920, 1496, 1472, 1270, 1132, 1089, 1008, 818, 672 $cm^{-1}$ |
| 71 | n-Butyl | H | 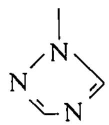 | 2 | n-Pentyl | 69–71 |
| 72 | $H_3C—$ | $H_3C—$ | 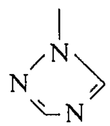 | 0 | 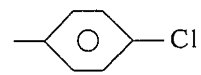 | 76–79 |
| 73 | 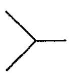 | H | 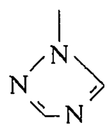 | 0 | 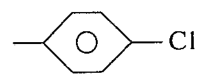 | IR (Film): 2955, 1494, 1471, 1279, 1130, 1050, 1007, 815, 673 $cm^{-1}$ |

(Fortsetzung)

| Verb. | $R^1$ | $R^2$ | Az (Salz) | n | $R^3$ | Schmp. (°C) |
|---|---|---|---|---|---|---|
| 74 | Äthyl | H | 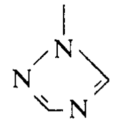 | 0 | n-Propyl | IR (Film): 2960, 2925, 1495, 1270, 1192, 1133, 1003, 817, 675 $cm^{-1}$ |
| 75 | n-Butyl | H | 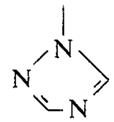 | 0 | n-Pentyl | IR (Film): 2955, 2925, 2860, 1496, 1460, 1271, 1190, 1133, 1008, 677 $cm^{-1}$ |
| 76 | n-Pentyl | H | 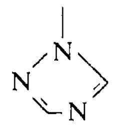 | 0 | n-Hexyl | IR (Film): 2955, 2925, 2860, 1497, 1460, 1272, 1192, 1135, 1012, 678 $cm^{-1}$ |
| 77 | H— | H | 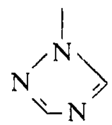 | 0 | 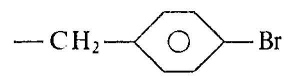 | 46–49 |
| 78 | H— | H | 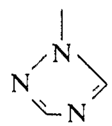 | 0 | 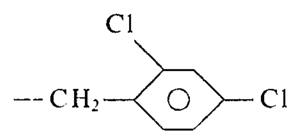 | 68–70 |
| 79 | H— | H | 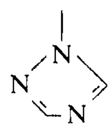 | 0 | 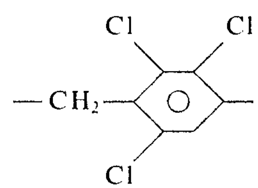 | 60–63 |

(Fortsetzung)

| Verb. | $R^1$ | $R^2$ | Az (Salz) | n | $R^3$ | Schmp. ( C) |
|---|---|---|---|---|---|---|
| 80 | 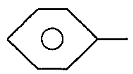 | H | 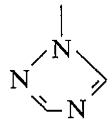 | 0 | 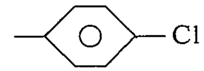 | 100–103 |
| 81 | 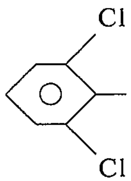 | H | 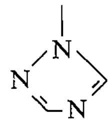 | 1 | tert.-Butyl | 115–116*) |
| 82 | 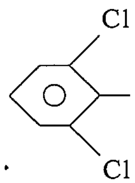 | H | 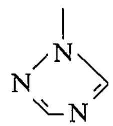 | 2 | tert.-Butyl | 164–165 |
| 83 | n-Butyl | H | 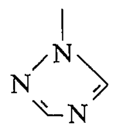 | 0 | tert.-Butyl | IR (Film): 2958, 1492, 1459, 1364, 1272, 1190, 1158, 1134, 1010, 677 $cm^{-1}$ |
| 84 | i-Butyl | H | 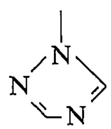 | 0 | tert.-Butyl | IR (Film): 2952, 2862, 1494, 1457, 1364, 1270, 1189, 1157, 1132, 1007, 675 $cm^{-1}$ |

*) Diastereomerengemisch

(Fortsetzung)

| Verb. | $R^1$ | $R^2$ | Az (Salz) | n | $R^3$ | Schmp. (°C) |
|---|---|---|---|---|---|---|
| 85 | i-Butyl | H | 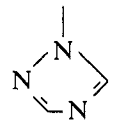 | 0 | 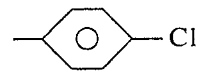 | 45–46 |
| 86 | i-Butyl | H | 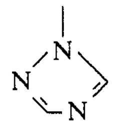 | 1 | 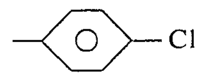 | 145–112 |
| 87 | i-Butyl | H | 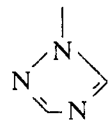 | 2 | 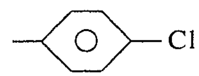 | 140–141 |
| 88 | 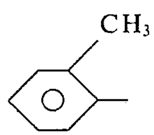 | H | 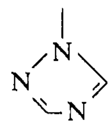 | 0 | tert.-Butyl | 98–101 |
| 89 | 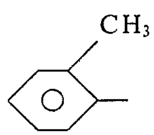 | H | 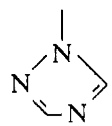 | 0 | tert.-Butyl | 93–95 |
| 90 | 3-Heptyl | H | 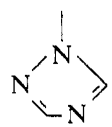 | 0 | 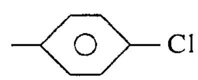 | IR (Film): 2954, 2922, 1472, 1269, 1131, 1088, 1008, 819, 673 $cm^{-1}$ |

(Fortsetzung)

| Verb. | $R^1$ | $R^2$ | Az (Salz) | n | $R^3$ | Schmp. (°C) |
|---|---|---|---|---|---|---|
| 91 | 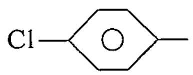 | H | 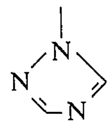 | 0 | 3-Methylbutyl | IR (Film):<br>2962, 1489, 1271, 1130,<br>1088, 1012, 676 $cm^{-1}$ |
| 92 | 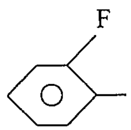 | H | 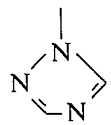 | 0 | 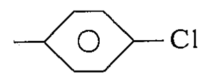 | 105–107 |
| 93 | 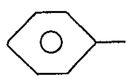 | H | 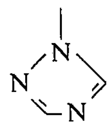 | 0 | tert.-Butyl | 106–108 |
| 94 | 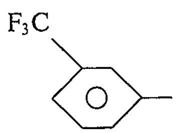 | H | 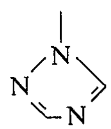 | 0 | tert.-Butyl | 40–43 |
| 95 | 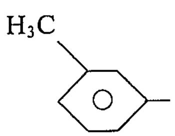 | H | 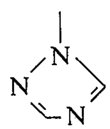 | 0 | tert.-Butyl | 85–87 |
| 96 | 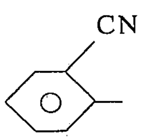 | H | 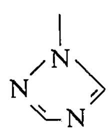 | 0 | tert.-Butyl | 110–112 |

(Fortsetzung)

| Verb. | $R^1$ | $R^2$ | Az (Salz) | n | $R^3$ | Schmp. (°C) |
|---|---|---|---|---|---|---|
| 97 | 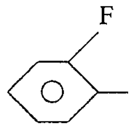 | H | 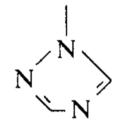 | 0 | tert.-Butyl | 94–95 |
| 98 | 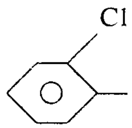 | H | 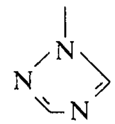 | 0 | Ethyl | 59–61 |
| 99 | 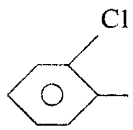 | H | 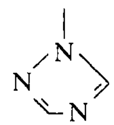 | 2 | Ethyl | 103–104 |
| 100 | 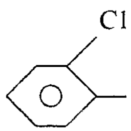 | H | 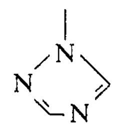 | 0 | n-Butyl | IR (Film): 2958, 2928, 1494, 1274, 1132, 1008, 748, 678 $cm^{-1}$ |
| 101 | 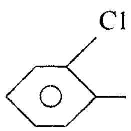 | H | 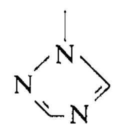 | 0 | n-Hexyl | IR (Film): 2955, 2923, 1491, 1271, 1129, 1007, 744, 675 $cm^{-1}$ |
| 102 | 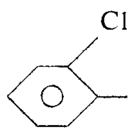 | H | 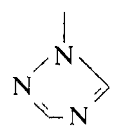 | 0 | $-CH_2-$ 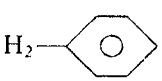 | 96–98 |

(Fortsetzung)

| Verb. | $R^1$ | $R^2$ | Az (Salz) | n | $R^3$ | Schmp. ( C) |
|---|---|---|---|---|---|---|
| 103 | 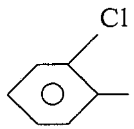 | H | 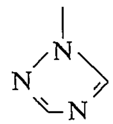 | 2 | 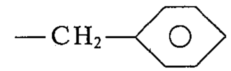 | 130–133 |
| 104 | 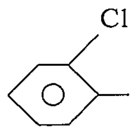 | H | 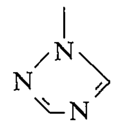 | 0 | 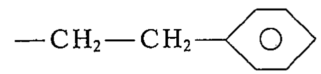 | 58–61 |
| 105 | 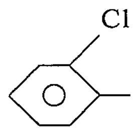 | H | 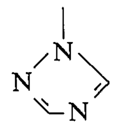 | 2 | 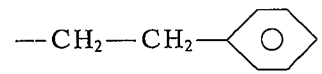 | 129–130 |
| 106 | 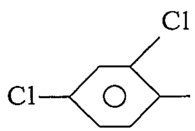 | H | 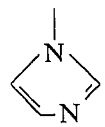 | 0 | 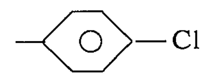 | 108–110 |
| 107 | H | H | 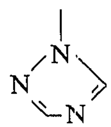 | 0 | 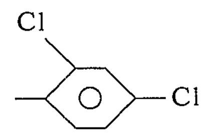 | 84–87 |
| 108 | H | H | 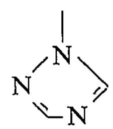 | 1 | 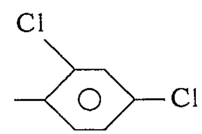 | 152–155 |

(Fortsetzung)

| Verb. | $R^1$ | $R^2$ | Az (Salz) | n | $R^3$ | Schmp. (°C) |
|---|---|---|---|---|---|---|
| 109 | H | H | 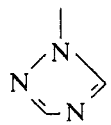 | 2 | 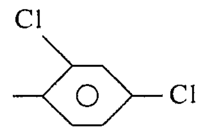 | |
| 110 | 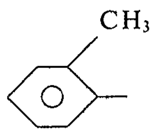 | H | 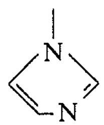 · HCl | 0 | tert.-Butyl | 168–170 |
| 111 | 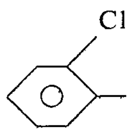 | H | 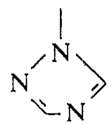 · HCl | 0 | n-Butyl | 136–139 |
| 112 | 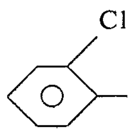 | H | 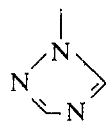 · HCl | 0 | 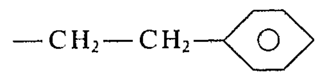 | 145–155 |
| 113 | 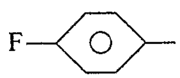 | H | 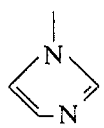 | 0 | 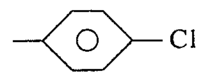 | 90–92 |
| 114 | 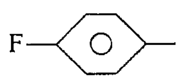 | H | 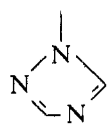 | 0 | 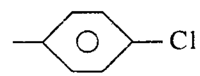 | 119–120 |
| 115 | 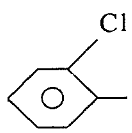 | H | 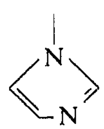 | 0 | 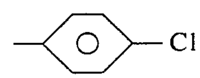 | 69–71 |

(Fortsetzung)

| Verb. | $R^1$ | $R^2$ | Az (Salz) | n | $R^3$ | Schmp. (°C) |
|---|---|---|---|---|---|---|
| 116 | 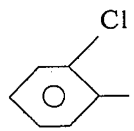 | H | 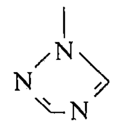 | 0 | 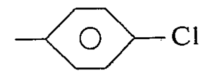 | 134–136 |
| 117 | 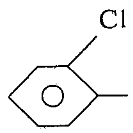 | H | 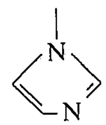 | 0 | tert.-Butyl | IR (Film): 3098, 2950, 1463, 1211, 1158, 1002, 1040, 739, 657 $cm^{-1}$ |
| 118 | 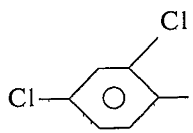 | H | 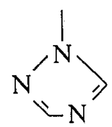 | 2 | tert.-Butyl | 940 |
| 119 | 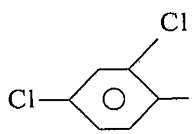 | H | 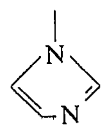 | 2 | tert.-Butyl | 153–156 |
| 120 | 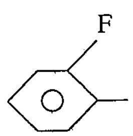 | H | 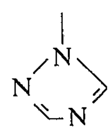 | 2 | tert.-Butyl | 143–145 |
| 121 | 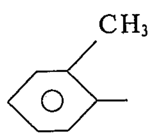 | H | 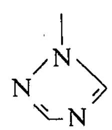 | 2 | tert.-Butyl | 160–162 |

(Fortsetzung)

| Verb. | $R^1$ | $R^2$ | Az (Salz) | n | $R^3$ | Schmp. (°C) |
|---|---|---|---|---|---|---|
| 122 | 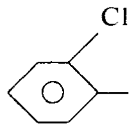 | H | 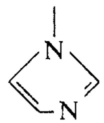 | 2 | tert.-Butyl | 113–116 |
| 123 | 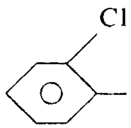 | H | 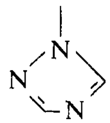 | 2 | 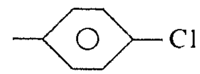 | 155–158 |
| 124 | 3-Heptyl | H | 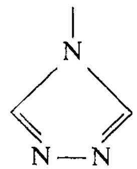 | 0 | 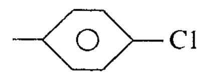 | IR (Film): 2956, 2922, 1472, 1468, 1168, 1090, 1009, 817, 647 $cm^{-1}$ |
| 125 | 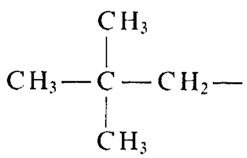 | H | 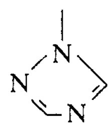 | 0 | 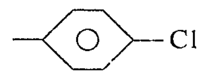 | 83–86 |
| 126 | 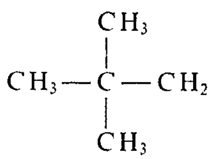 | H | 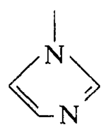 | 0 | 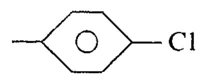 | 47–51 |
| 127 | 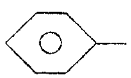 | H | 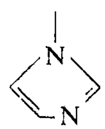 | 0 | 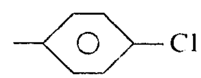 | 89–90 |
| 128 | 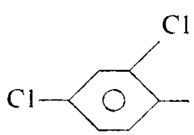 | H | 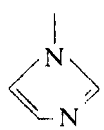 | 0 | 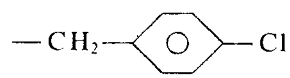 | IR (Film): 3103, 1586, 1468, 1378, 1217, 1090, 1067, 1048, 1012, 825, 659 $cm^{-1}$ |

Die neuen α-Azolylsulfide, α-Azolyl-sulfoxide und α-Azolyl-sulfone und ihre Salze zeigen eine erheblich breitere fungizide Wirkung und eine überlegene Pflanzenverträglichkeit als das bekannte 1-[2,4-Dichlorphenyl-β-allyläthyläther]-imidazol. Die neuen Wirkstoffe können auch in Form ihrer Salze, z. B. Hydrochloride, Oxalate oder Nitrate verwendet werden.

Von großem Interesse sind die erfindungsgemäßen fungiziden Mittel bei Pilzerkrankungen an verschiedenen Kulturpflanzen, z. B. bei

Ustilago scitaminea (Zuckerrohrbrand),
Hemileia vastatrix (Kaffeerost),
Uromyces fabae bzw. appendiculatus (Bohenrost),
Puccinia Arten (Getreiderost),
Erysiphe graminis (Getreidemehltau),
Botrytis cinerea an Rebe, Erdbeeren,
Unicula necator,
Sphaerotheca fuliginea,
Erysiphe cichoracearum,
Podosphaera leucotricha.

Unter Kulturpflanzen verstehen wir in diesem Zusammenhang insbesondere Weizen, Roggen, Gerste, Hafer, Reis, Mais, Apfelbaum, Gurken, Bohnen, Kaffee, Zuckerrohr, Weinrebe, Erdbeeren sowie Zierpflanzen im Gartenbau.

Die erfindungsgemäßen Wirkstoffe sind systemisch wirksam. Die systemische Wirksamkeit dieser Mittel ist von besonderem Interesse im Zusammenhang mit der Bekämpfung von inneren Pflanzenkrankheiten, z. B. Getreiderost, Getreidemehltau.

Die erfindungsgemäßen Mittel können gleichzeitig das Wachstum von zwei oder mehr der genannten Pilze unterdrücken und besitzen eine hohe Pflanzenverträglichkeit. Die zur Bekämpfung der phytopathogenen Pilze erforderlichen Aufwandmengen liegen zwischen 0,05 und 2 kg Wirkstoff/ha Kulturfläche.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z. B. durch Vermischen des Wirkstoffes mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel, wie Aromaten (z. B. Xylol, Benzol), chlorierte Armaten (z. B. Chlorbenzole), Paraffine (z. B. Erdölfraktionen), Alkohole (z. B. Methanol, Butanol), Amine (z. B. Äthanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel, wie nichtionogene und anionische Emulgatoren (z. B. Polyoxyäthylen-Fettalkohol-Äther, Alkylsulfonate und Arylsulfonate) und Dispergiermittel, wie Lignin, Sulfitablaugen und Methylcellulose.

Die Formulierungen enthalten im allgemeinen zwischen 0,1 und 95 Gewichtsprozent Wirkstoff, vorzugsweise zwischen 0,5 und 90%. Die Formulierungen bzw. die daraus hergestellten gebrauchsfertigen Zubereitungen, wie Lösungen, Emulsionen, Suspensionen, Pulver, Pasten oder Granulate, werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, z. B. Herbiziden, Insektiziden, Wachstumsregulatoren und Fungiziden oder auch mit Düngemitteln vermischt werden.

Für die folgenden Versuche, die die fungizide Wirksamkeit der erfindungsgemäßen Verbindungen belegen, wurde zu Vergleichszwecken der folgende bekannte Wirkstoff verwendet

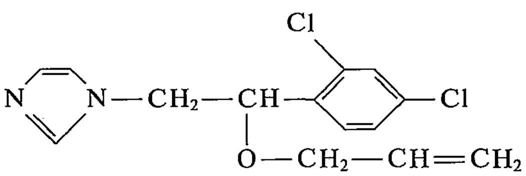

(A)

bekannt aus DE-OS 20 63 857

Beispiel 14
Gerstenmehltau

Blätter von in Töpfen gewachsenen Gerstenkeimlingen der Sorte »Firlbecks Union« werden mit wäßrigen Emulsionen aus 80% (Gew.-%) Wirkstoff und 20% Emulgiermittel besprüht und nach dem

Antrocknen des Spritzbelages mit Oidien (Sporen) des Gerstenmehltaus (Erysiphe graminis var. hordei) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

0 = kein Befall, abgestuft bis 5 = Totalbefall

| Wirkstoff | Befall der Blätter nach Spritzungen mit . . .%iger Wirkstoffbrühe | |
|---|---|---|
| | 0,05 | 0,025 |
| 4 | 2 | — |
| 6 | 0 | 3 |
| 9 | 0 | 3 |
| 10 | 0 | 4 |
| 12 | 0 | 2 |
| 14 | 0 | 2 |
| 15 | 0 | 3 |
| 18 | 0 | 3 |
| 21 | 0 | 2–3 |
| 25 | 0 | 2 |
| 26 | 0 | 3 |
| 27 | 0 | 3 |
| 30 | 0 | 0 |
| 31 | 0 | 3 |
| 40 | 2 | — |
| 41 | 0 | 2 |
| 48 | 0 | 2 |
| 50 | 0 | 3 |
| 54 | 0 | 2–3 |
| 59 | 0 | 2 |
| 64 | 0 | 2 |
| 65 | 0 | 3 |
| 7 | 0 | 0 |
| 80 | 0 | 0 |
| 85 | 0 | 0 |
| 90 | 0 | 0 |
| 92 | 0 | 0 |

0 = kein Befall, abgestuft bis 5 = Totalbefall

| Wirkstoff | Befall der Blätter nach Spritzungen mit . . .%iger Wirkstoffbrühe | |
|---|---|---|
| | 0,05 | 0,025 |
| 94 | 0 | 0 |
| 104 | 0 | 0 |
| 113 | 0 | 0 |
| 114 | 0 | 0 |
| 115 | 0 | 0 |
| 116 | 0 | 0 |
| 125 | 0 | 0 |
| 126 | 0 | 0 |
| 127 | 0 | 0 |
| 128 | 0 | 0 |
| Kontrolle (unbehandelt) | 5 | |

Beispiel 15
Weizenmehltau

Entsprechend wie in Beispiel 9 beschrieben, werden Blätter, von in Töpfen gewachsenen Weizenkeimlingen der Sorte »Jubilar« behandelt und mit Oidien (Sporen) des Weizenmehltaus (Erysiphe graminis var. tritici) infiziert und im übrigen wie in Beispiel 9 behandelt.

| Wirkstoff | Befall der Blätter nach Spritzungen mit . . .%iger Wirkstoffbrühe 0,05 | 0,025 |
|---|---|---|
| 4 | 0 | 2 |
| 30 | 0 | 0 |
| 40 | 0 | 2 |
| 80 | 0 | 0 |
| 85 | 0 | 0 |
| 90 | 0 | 0 |
| 92 | 0 | 0 |
| 94 | 0 | 0 |
| 104 | 0 | 0 |
| 113 | 0 | 0 |
| 114 | 0 | 0 |
| 115 | 0 | 0 |
| 116 | 0 | 0 |
| 125 | 0 | 0 |
| 126 | 0 | 0 |
| 127 | 0 | 1 |
| 128 | 0 | 2 |
| A | 1 | 3 |
| Kontrolle (unbehandelt) | 5 | |

Beispiel 16
Weizenbraunrost

Blätter von in Töpfen gewachsenen Weizenkeimlingen der Sorte »Jubilar« werden 24 Stunden vor der Spritzung künstlich mit Sporen des Weizenbraunrostes (Puccinia recondita) infiziert und bei 20 bis 25° C in einer wasserdampfgesättigten Kammer aufgestellt. Danach werden die Pflanzen mit wäßrigen Emulsionen aus 80% (Gewichtsprozent) Wirkstoff und 20% Emulgiermittel besprüht. Nach dem Antrocknen des Spritzbelages werden die Versuchspflanzen im Gewächshaus bei Temperaturen zwischen 20 und 22° C und 75 bis 80% relativer Luftfeuchtigkeit aufgestellt. Nach 10 Tagen wird das Ausmaß der Rostpilzentwicklung ermittelt.

| Wirkstoff | Befall der Blätter nach Spritzungen mit 0,05%iger Wirkstoffbrühe |
|---|---|
| 4 | 2 |
| 12 | 0 |
| 27 | 0 |
| 30 | 0 |
| 7 | 0 |
| 80 | 0 |
| 85 | 0 |
| 90 | 0 |
| 92 | 0 |
| 104 | 0 |
| 113 | 0 |
| 114 | 0 |
| 116 | 0 |
| 125 | 0 |
| 126 | 0 |
| 127 | 0 |
| A | 3 |
| Kontrolle (unbehandelt) | 5 |

Beispiel 17

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 18

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 19

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 20

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 21

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 22

3 Gewichtsteile der Verbindung 3 werden mit 97 Gewichtsteilen feinteiligem Kaolin innig vermischt. Man erhält auf diese Weise ein Stäubmittel, das 3 Gewichtsprozent des Wirkstoffs enthält.

Beispiel 23

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 24

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 25

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche**

1. α-Azolylsulfide, -sulfoxide und -sulfone der Formel

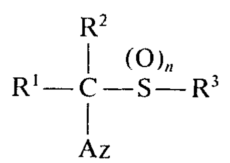

in der

$R^1$ Wasserstoff, Alkyl, Alkoxycarbonyl oder gegebenenfalls substituiertes Aryl,
$R^2$ Wasserstoff oder Alkyl,
$R^3$ Alkyl, Alkenyl, Alkinyl, gegebenenfalls substituiertes Phenyl oder gegebenenfalls substituiertes Aralkyl,
Az Imidazol-1-yl, Pyrazol-1-yl, 1,2,4-Triazol-1-yl, 1,2,4-Triazol-4-yl, Tetrazol-1-yl oder Tetrazol-2-yl und
n 0, 1 oder 2 bedeuten
sowie deren Salze oder Metallkomplexe.

2. Mittel zur Bekämpfung von Pilzen, dadurch gekennzeichnet, daß sie eine Verbindung nach Anspruch 1 enthalten.

3. Verfahren zur Bekämpfung von Pilzen, dadurch gekennzeichnet, daß man den Boden oder die Pflanzen behandelt mit einer Verbindung nach Anspruch 1.

4. Verfahren zur Herstellung einer Verbindung nach Anspruch 1, daß man α-Chlorsulfide der Formel

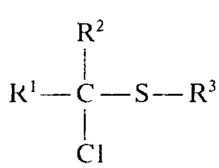

in welcher
$R^1$, $R^2$ und $R^3$ die im Anspruch 1 angegebene Bedeutung haben, mit Azolen H—Az, in denen Az die im Anspruch 1 angegebene Bedeutung hat, ggf. in Gegenwart einer Base und ggf. in Gegenwart eines Verdünnungsmittels umsetzt und die so erhaltenen α-Azolylsulfide ggf. mit Säure in ihre Salze oder ggf. mit Metallsalzen in ihre Metallkomplexe oder ggf. mit geeigneten Oxidationsmitteln in die α-Azolylsulfone überführt.

5. 4-Chlorphenyl-[(2-chlorphenyl)-1,2,4-triazol-1-yl-methyl]-sulfid.
6. 4-Chlorphenyl-[(4-fluorphenyl)-1,2,4-triazol-1-yl-methyl]-sulfid.
7. 4-Chlorphenyl-[(phenyl)-imidazol-1-yl-methyl]-sulfid.
8. 4-Chlorphenyl-[1(1,2,4-triazol-1-yl)-3,3-dimethylbutyl]-sulfid.
9. 4-Chlorphenyl-[1(imidazol-1-yl)-3,3-dimethylbutyl]-sulfid.
10. Mittel zur Bekämpfung von Pilzen, enthaltend ein α-Azolylsulfid gemäß den Ansprüchen 5 bis 9.

**Claims**

1. α-azolyl sulfides, sulfoxides and sulfones of the formula

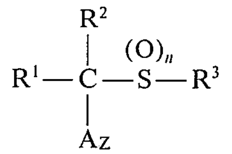

where $R^1$ denotes hydrogen, alkyl, alkoxycarbonyl, or unsubstituted or substituted aryl, $R^2$ denotes hydrogen or alkyl, $R^3$ denotes alkyl, alkenyl, alkynyl, unsubstituted or substituted phenyl, or unsubstituted or substituted aralkyl, Az denotes imidazol-1-yl, pyrazol-1-yl, 1,2,4-triazol-1-yl, 1,2,4-triazol-4-yl, tetrazol-1-yl or tetrazol-2-yl, and n denotes one of the integers 0, 1 and 2, and their salts and metal complexes.

2. Agents for combatting fungi, characterized in that they contain a compound as claimed in claim 1.

3. A process for combatting fungi, characterized in that the soil or the plants are treated with a compound as claimed in claim 1.

4. A process for the manufacture of a compound as claimed in claim 1, characterized in that α-chlorosulfides of the formula

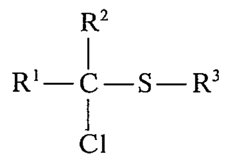

where $R^1$, $R^2$ and $R^3$ have the meanings given in claim 1, are reacted with azoles H—Az, where Az has the meaning given in claim 1, in the presence or absence of a base and in the presence or absence of a diluent and the resulting α-azolylsulfides may be converted with acid into their salts, with metal salts into their metal complexes, or with suitable oxidants into α-azolylsulfones.

5. 4-Chlorophenyl-[(2-chlorophenyl)-1,2,4-triazol-1-yl-methyl]-sulfide.
6. 4-Chlorophenyl-[(4-fluorophenyl)-1,2,4-triazol-1-yl-methyl]-sulfide.
7. 4-Chlorophenyl-[(phenyl)-imidazol-1-yl-methyl]-sulfide.
8. 4-Chlorophenyl-[1-(1,2,4-triazol-1-yl)-3,3-dimethylbutyl]-sulfide.
9. 4-Chlorophenyl-[1-(imidazol-1-yl)-3,3-dimethylbutyl]-sulfide.
10. Agents for combatting fungi, containing an α-azolylsulfide as claimed in any of claims 5 to 9.

**Revendications**

1. α-azolylsulfures, -sulfoxydes et -sulfones de formule

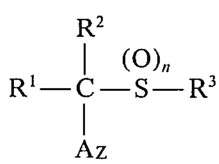

dans laquelle
$R^1$ est de l'hydrogène, un reste alkyle, alkoxycarbonyle ou un reste aryle éventuellement substitué,
$R^2$ est de l'hydrogène ou un reste alkyle,
$R^3$ est un reste alkyle, alkényle, alkynyle, un reste phényle éventuellement substitué ou un reste aralkyle éventuellement substitué,

Az est un reste imidazol-1-yle, pyrazol-1-yle, 1,2,4-triazol-1-yle, 1,2,4-triazol-4-yle, tétrazol-1-yle ou tétrazol-1-yle, et

n vaut 0, 1 ou 2,

ainsi que les sels ou les complexes métalliques de ces composés.

2. Produits de lutte contre les champignons, caractérisés par le fait qu'ils contiennent un composé selon la revendication 1.

3. Procédé de lutte contre les champignons, caractérisé par le fait qu'on traite le sol ou les plantes avec un composé selon la revendication 1.

4. Procédé de préparation d'un composé selon la revendication 1, caractérisé par le fait qu'on fait réagir des $\alpha$-chlorosulfures de formule

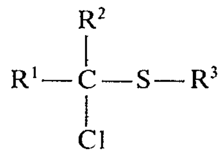

dans laquelle $R^1$, $R^2$ et $R^3$ ont la signification indiquée dans la revendication 1, avec des azoles H—Az, Az ayant la signification indiquée dans la revendication 1, le cas échéant en présence d'une base et, le cas échéant, en présence d'un diluant, et on transforme les $\alpha$-azolylsulfures ainsi obtenus le cas échéant en leurs sels avec des acides ou, le cas échéant, en leurs complexes métalliques avec des sels métalliques ou, le cas échéant, en $\alpha$-azolylsulfones avec des oxydants appropriés.

5. Sulfure de 4-chlorophényl-[(2-chlorophényl)-1,2,4-triazol-1-yl-méthyle].

6. Sulfure de 4-chlorophényl-[(4-fluorophényl)-1,2,4-triazol-1-yl-méthyle].

7. Sulfure de 4-chlorophényl-[(phényl)-imidazol-1-yl-méthyle].

8. Sulfure de 4-chlorophényl-[1-(1,2,4-triazol-1-yl)-3,3-diméthylbutyle].

9. Sulfure de 4-chlorophényl-[1-(imidazol-1-yl)-3,3-diméthylbutyle].

10. Produits de lutte contre les champignons, contenant un $\alpha$-azolylsulfure selon l'une des revendications 5 à 9.